# EUROPEAN PATENT APPLICATION

(11) **EP 4 249 035 A1**
(43) Date of publication of application: **27.09.2023**
(21) Application number: 21894956.8
(22) Date of filing: 02.11.2021
(51) Int. Cl.: A61M 37/00, A61K 9/00

(54) **MICROSTRUCTURE CAPABLE OF SELF-INTERLOCKING**

(30) Priority: 19.11.2020 KR 20200155987; 05.08.2021 KR 20210102890
(71) Applicant: Cursus Bio Inc., Seoul 06170 (KR)
(72) Inventor: KIM, Yonghee, Seoul 04763 (KR); FAKHRAEI LAHIJI, Shayan, Seoul 04763 (KR)
(74) Representative: Frenkel, Matthias Alexander
(86) International application number: PCT/KR2021/015704
(87) International publication number: WO 2022/108185

(57) **Abstract**

A microstructure is disclosed. The microstructure comprises a base film and a plurality of microneedles formed on one surface of the base film, wherein the microneedles may each comprise: a needle body; and support wings which are disposed in a plurality around the needle body, connect the outer surface of the needle body and the base film, and have a smaller thickness than the needle body.

## Description

### [Technical Field]

The present invention relates to a microstructure, and more particularly, to a microstructure capable of penetrating the skin and supplying a drug.

### [Background Art]

There are oral, injectable, transdermal, and other administration routes for delivering drugs to the body. Oral administration is convenient administration that can increase patient drug compliance, and active ingredients are delivered to the body in the form of capsules, tablets, and syrups. However, the active ingredients may be inactivated due to first-pass metabolism or the like in the liver, and the absorption rate of biopharmaceuticals is actually relatively low. Therefore, in order to express accurate and rapid medicinal effects of drugs, therapeutic agents, etc., they are administered to the human body in an injectable form by piercing the skin barrier. When they are delivered in the injectable form, it has an advantage that the activity of the active ingredients is maintained, but has disadvantages such as risk of infection, inaccurate dose administration, fear, and pain.

Various microstructured transdermal drug delivery systems, including minimally penetrating microneedles, have been developed in order to overcome the limitations of existing oral and injectable routes of administration. Microstructures are mainly manufactured in biodegradable/dissolving, solid, coating, and hollow forms. Biodegradable microstructures are transdermal delivery systems in which various materials including polymers and active ingredients (API/cosmetics or pharmaceuticals) are formulated in the form of fine needles and inserted into the skin, and then the loaded materials are dissolved by body fluids so that drugs can be delivered without pain.

Existing microstructures are composed of a wide base film and microneedles with a thin body, and the microneedles are mainly provided in the shape of a cone or a polygonal pyramid. The shape of such microneedles has a limitation in that it is not easy to deliver a drug in a fixed quantity due to the high possibility of detachment after insertion into the skin. In addition, since the microneedles are manufactured in a fine size of tens to hundreds of microns and the drug load amount is limited, it has a limitation in that it is mainly applied to the cosmetic field because it cannot deliver the desired amount of the active ingredients to the body. In addition, as the microstructure does not maintain a state of close contact with the skin, it takes a long time for dissolution of the drug, and due to this, it has limitations such as the risk of causing various skin diseases including itching, dermatitis, and allergy, and uncomfortable attachment. In addition, when the microstructure is separated from the mold or pressed with a finger so that it is inserted into the skin, there is a limitation in that the microneedles are broken or cannot be accurately inserted into the skin depending on the force action direction and magnitude.

### [Disclosure]

### [Technical Problem]

The present invention provides a microstructure capable of quantitative drug delivery to the skin.

### [Technical Solution]

A microstructure according to the present invention may include: a base film; and a plurality of microneedles formed on one surface of the base film, wherein the microneedle may include: a needle body; and support wings which are disposed in a plurality around the needle body, connect the outer surface of the needle body and the base film, and have a thickness thinner than that of the needle body.

In addition, the support wings may be symmetrical about the center of the needle body.

In addition, the support wings may gradually decrease in thickness as they are away from the center of the needle body.

Further, the needle body may include: a first region coupled to the base film; a second region extending from the first region and gradually increasing in width as the distance from the first region increases; and a third region extending from the second region and gradually decreasing in width as it goes to an end thereof, and each of the support wings may be provided in a section between the first region and the second region.

In addition, the region where the support wings and the base film are connected may have an end located inside the maximum radius region of the second region when viewed from above.

In addition, the region where the support wings and the base film are connected may have an end located on the same line as the maximum radius region of the second region when viewed from above.

In addition, the region where the support wings and the base film are connected may have an end located outside the maximum radius region of the second region when viewed from above.

In addition, the connection region between the second region and the third region may have an outer circumferential surface provided as a curved surface.

Further, the needle body includes: a first region coupled to the base film; a second region extending from the first region and gradually increasing in width as the distance from the first region increases; and a third region extending from the second region and gradually decreasing in width as it goes to an end thereof, and each of the support wings may extend downward from an end of the third region and may be connected to the base film.

In addition, the connection region where the support wings and the base film are connected may be larger than a maximum radius of the second region.

Further, the support wings may include: a first support wing 250 located on one side of the needle body; and a second support wing located on the opposite side of the first support wing centering around the needle body, and the first support wing and the second support wing may have different thicknesses.

In addition, the support wings may gradually increase in thickness as it goes from an upper end thereof to a lower end adjacent to the base film.

### [Advantageous Effects]

According to the present invention, the microneedles are provided in a structure in which a needle body and support wings are coupled, and since self-interlocking is possible, a state in which the microstructure is in close contact with the skin can be stably maintained. Due to this, the microstructure is dissolved and can penetrate the skin, thereby enabling quantitative drug delivery. In addition, since the support wings connect the needle body and the base film, breakage of the microneedles can be prevented in the process of being separated from the mold or penetrating into the skin. In addition, since the needle body consists of a first region, a second region having a maximum radius region, and a third region where a tip is formed, a sufficient amount of drug can be loaded.

### [Description of Drawings]

FIG. 1 is a perspective view showing a microstructure according to one embodiment of the present invention.
FIG. 2 is an enlarged view of the microneedle shown in FIG. 1.
FIG. 3 is a view showing an optimized size of the microneedle according to the embodiment of FIG. 2.
FIG. 4 is a perspective view showing a microstructure according to other embodiment of the present invention.
FIG. 5 is an enlarged view of the microneedle shown in FIG. 4.
FIG. 6 is a view showing a microneedle according to another embodiment of the present invention.
FIG. 7 is a view showing a microneedle according to another embodiment of the present invention.
FIG. 8 is a view showing a microneedle according to another embodiment of the present invention.
FIG. 9 is a view showing a process of manufacturing a microstructure using a mold.
FIG. 10 is a view showing a process of inserting a microstructure according to an embodiment of the present invention into the skin.
FIG. 11 is a view showing a microneedle according to another embodiment of the present invention.
FIG. 12 is a view showing a microneedle according to another embodiment of the present invention.
FIGS. 13 and 14 are views showing various shapes of a needle body according to an embodiment of the present invention.
FIG. 15 is a view showing a needle body and a base film according to one embodiment of the present invention.
FIG. 16 is a view showing cross sections of support wings according to various embodiments of the present invention.
FIG. 17 is a cross-sectional view showing the arrangement of support wings according to various embodiments of the present invention.
FIG. 18 is an image showing a microstructure and microneedles fabricated according to an embodiment of the present invention.
FIG. 19 is a view showing a process of injecting a drug by penetrating the microstructure according to an embodiment of the present invention into the skin.
FIG. 20 is a view showing a process of injecting a drug by penetrating a microstructure according to a comparative example into the skin.
FIG. 21 is a view showing a microstructure fabricated according to one embodiment of the present invention and microneedles thereof.
FIG. 22 is a view showing a microstructure fabricated according to other embodiment of the present invention and microneedles thereof.
FIG. 23 is an enlarged view of a microneedle fabricated according to an embodiment of the present invention.

### [Best Mode for Carrying Out the Invention]

The microstructure according to the present invention may include: a base film; and a plurality of microneedles formed on one surface of the base film, wherein the microneedles may include: a needle body; and support wings which are disposed in a plurality around the needle body, connect the outer surface of the needle body and the base film, and have a thickness thinner than that of the needle body.

### [Mode for Carrying Out the Invention]

Hereinafter, preferred embodiments of the present invention will be described in detail with reference to the accompanying drawings. However, the technical idea of the present invention is not limited to the embodiments described herein and may be embodied in other forms. Rather, the embodiments introduced herein are provided so that the disclosed content will be thorough and complete, and the spirit of the present invention will be sufficiently conveyed to those skilled in the art.

In the present specification, when an element is referred to as being on another element, it means that it may be directly formed on the other element or a third element may be interposed therebetween. Also, in the drawings, the thicknesses of films and regions are exaggerated for effective explanation of technical content.

In addition, although terms such as first, second, and third have been used in order to describe various elements in various embodiments of the present specification, these elements should not be limited by these terms. These terms have only been used in order to distinguish one element from another. Therefore, what is referred to as a first element in one embodiment may be referred to as a second element in another embodiment. Each embodiment described and exemplified herein also includes its complementary embodiments. In addition, in the present specification, 'and/or' has been used to mean including at least one of the elements listed before and after.

In the specification, expressions in the singular number include plural expressions unless the context clearly dictates otherwise. In addition, the terms "comprise" or "having" are intended to designate that a feature, number, step, component, or a combination thereof described in the specification exists, but should not be understood as excluding the possibility of the presence or addition of one or more other features, numbers, steps, components, or combinations thereof. In addition, in the present specification, "connection" is used as a meaning including both indirectly and directly connecting a plurality of components.

In addition, in the following description of the present invention, if it is determined that a detailed description of a related known function or configuration may unnecessarily obscure the subject matter of the present invention, the detailed description will be omitted.

The microstructure according to various embodiments of the present invention may be loaded with a drug and deliver the drug through the skin of the body.

Drugs mean a concept in a broad sense, and include not only therapeutic agents for treatment purpose in a narrow sense, but also all of energy, nano-components, beauty ingredients (e.g., anti-wrinkle agents, skin aging inhibitors, and skin whitening agents), cell culture solutions, and the like.

Specifically, the therapeutic agents include chemical drugs, protein/peptide drugs, peptide drugs, nucleic acid molecules for gene therapy, and the like.

For example, the therapeutic agents may include anti-inflammatory drugs, analgesics, anti-arthritic drugs, antispasmodics, anti-depressants, antipsychotic drugs, tranquilizers, anti-anxiety drugs, narcotic antagonists, anti-parkinsonian drugs, cholinergic agonists, anti-cancer drugs, anti-angiogenic inhibitors, and immunosuppressive drugs. , antiviral drugs, antibiotics, appetite suppressants, analgesics, anticholinergics, antihistamines, antimigraine drugs, hormones, coronary vascular, cerebrovascular or peripheral vascular vasodilators, contraceptives, antithrombotic drugs, diuretics, antihypertensives, cardiovascular disease treatment, etc.

In particular, the protein/peptide drugs may include hormones, hormone analogues, enzymes, enzyme inhibitors, signal transduction proteins or parts thereof, antibodies or parts thereof, single-chain antibodies, binding proteins or binding domains thereof, antigens, attachment proteins, structural proteins, regulatory proteins, toxin proteins, cytokines, transcriptional regulatory factors, blood coagulation factors, vaccines, etc. More specifically, the protein/peptide drugs may include insulin, insulin-like growth factor 1 (IGF-1), growth hormone, erythropoietin, granulocyte-colony stimulating factors (G-CSFs), granulocyte/macrophagecolony stimulating factors (GM-CSFs), interferon alpha, interferon beta, interferon gamma, interleukin-1 alpha and beta, interleukin-3, interleukin-4, interleukin-6, interleukin-2, epidermal growth factors (EGFs), calcitonin, adrenocorticotropic hormone (ACTH), tumor necrosis factor (TNF), atobisban, buserelin, cetrorelix, deslorelin, desmopressin, dynorphin A (1-13), elcatonin, eleidosin, eptifibatide, growth hormone releasing hormone-II (GHRH-II), gonadorelin, goserelin, histrelin, leuprorelin, lypressin, octreotide, oxytocin, pitressin, secretin, sincalide, terlipressin, thymopentin, thymosin α1, triptorelin, bivalirudin, carbetocin, cyclosporine, exedine, lanreotide, luteinizing hormone-releasing hormone (LHRH), nafarelin, parathyroid hormone, pramlintide, enfuvirtide (T-20), thymalfasin, and ziconotide.

In addition, since the microstructure according to the present invention is self-interlocked after being inserted into skin tissue, it may be made of a biocompatible or biodegradable material. The biocompatible or biodegradable material, as a material that is substantially non-toxic to the human body, chemically inert, and non-immunogenic, has the advantage of being dissolved after finally being penetrated into the body.

The type of such a biocompatible material is not particularly limited, and examples thereof may include hyaluronic acid, polyester, polyhydroxyalkanoate (PHAs), poly(α-hydroxy acid), poly(β-hydroxy acid), poly(3-hydroxybutyrate-co-hydroxyvalerate (PHBV), poly(3-hydroxypropionate) (PHP), poly(3-hydroxyhexanoate) (PHH), poly(4-hydroxyacid), poly(4-hydroxybutyrate), poly(4-hydroxyvalerate), poly(4-hydroxyhexanoate), poly(ester amide)s, polycaprolactone, polylactide, polyglycolide, poly(lactide-co-glycolide) (PLGA), polydioxanone, polyorthoesters, polyetheresters, polyanhydrides, poly(glycolide-co-trimethylene carbonate), polyphosphoesters, polyphosphoester urethanes, poly(amino acid), polycyanoacrylates, poly(trimethylene carbonate), poly(imino carbonates), poly(tyrosine carbonate), polycarbonate, poly(tyrosine arylate), poly(alkylene oxalate), polyphosphagens, phytohemagglutinin-polyethylene glycol (PHA-PEG), ethylene vinyl alcohol copolymer (EVOH), polyurethane, silicone, polyester, polyolefin, polyisobutylene and ethylene-alpha olefin copolymer, styrene-isobutylene-styrene triblock copolymer, acrylic polymers and copolymers, vinyl halide polymers and copolymers, polyvinyl chloride, polyvinyl ether, polyvinyl methyl ether, polyvinylidene halide, polyvinylidene fluoride, polyvinylidene chloride, polyfluoroalkanes, polyperfluoroalkanes, polyacrylonitriles, polyvinyl ketones, polyvinylaromatics, polystyrenes, polyvinyl esters, polyvinyl acetates, ethylene-methyl methacrylate copolymers, acrylonitrile-styrene copolymers, ABS resin and ethylenevinyl acetate copolymer, polyamide, alkyd resin, polyoxymethylene, polyimide, polyether, polyacrylate, polymethacrylate, poly(acrylic acid-co-maleic acid), chitosan, dextran, cellulose, heparin, alginate, inulin, starch, or glycogen, or may include one or more selected from the group consisting of hyaluronic acid, polyester, polyhydroxyalkanoate (PHAs), poly(α-hydroxy acid), poly(β-hydroxy acid), poly(3-hydroxybutyrate-co-hydroxyvalerate (PHBV), poly(3-hydroxypropionate) (PHP), poly(3-hydroxyhexanoate) (PHH), poly(4-hydroxyacid), poly(4-hydroxybutyrate), poly(4-hydroxyvalerate), poly(4-hydroxyhexanoate), poly(ester amide)s, polycaprolactone, polylactide, polyglycolide, poly(lactide-co-glycolide) (PLGA), polydioxanone, polyorthoesters, polyetheresters, polyanhydrides, poly(glycolide-co-trimethylene carbonate), polyphosphoesters, polyphosphoester urethanes, poly(amino acid), polycyanoacrylates, poly(trimethylene carbonate), poly(imino carbonates), poly(tyrosine carbonate), polycarbonate, poly(tyrosine arylate), poly(alkylene oxalate), polyphosphagens, phytohemagglutinin-polyethylene glycol (PHA-PEG), chitosan, dextran, cellulose, heparin, alginate, inulin, starch, and glycogen.

When the microstructure is solid-type microneedles loaded with a biocompatible or biodegradable material, a drug may be additionally loaded.

Various embodiments of a microstructure capable of self-interlocking will be described below.

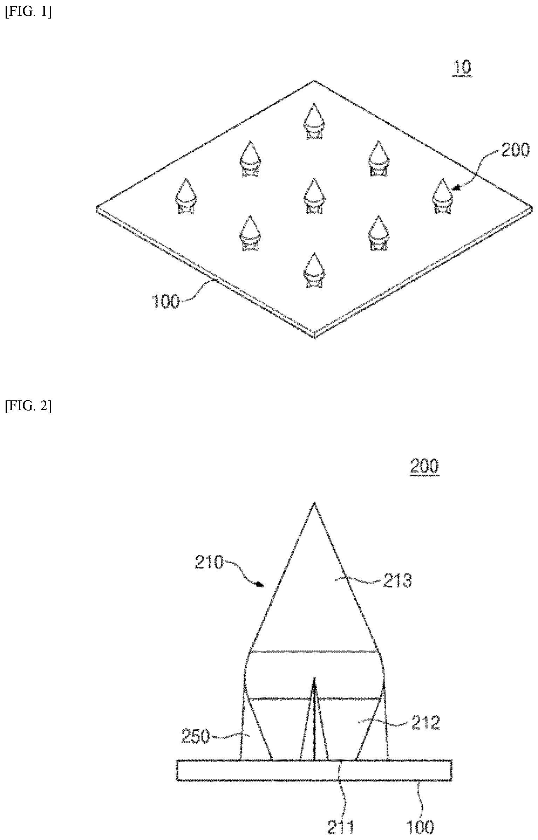

FIG. 1 is a perspective view showing a microstructure according to one embodiment of the present invention, and FIG. 2 is an enlarged view of the microneedle shown in FIG. 1.

Referring to FIGS. 1 and 2, the microstructure 10 includes a base film 100 and microneedles 200.

The base film 100 is a film with a thin thickness, and is provided at a predetermined width. The base film 100 may be provided as a film of a circular or polygonal shape.

A plurality of microneedles 200 are formed on one surface of the base film 100, and are provided in a structure capable of penetrating the skin and self-interlocking. The microneedle 200 includes a needle body 210 and support wings 250 .

The needle body 210 protrudes from one surface of the base film 100 to a predetermined height. Specifically, the needle body 210 has first to third regions 211 to 213. The first region 211 is a region coupled to one surface of the base film 100 and has a first width w1. The second region 212 extends from the first region 211 and gradually increases in width as the distance from the first region 211 increases. The maximum width of the cross section of the second region 212 has a second width w2 larger than the first width w1. The third region 213 extends from the second region 212, and gradually decreases in width as it goes toward an end thereof. The end of the third region 213 forms a sharp tip. The connection region 215 where the second region 212 and the third region 213 are connected has an outer circumferential surface provided as a curved surface.

A plurality of support wings 250 are formed at a thin thickness and are disposed to be spaced apart from each other along the circumference of the needle body 210. The support wings 250 may be disposed at the same angle between the needle body 210 as the center. The support wings 250 may be disposed symmetrically with each other around the needle body 210. In this embodiment, it will be described for example that four support wings 250 are formed, and are disposed at an angle of 90 degrees with respect to the needle body 210. However, the number and disposition of the support wings 250 are not limited thereto, and may be variously changed.

The support wings 250 connect the outer surface of the needle body 210 and the base film 100. The support wings 250 gradually decrease in thickness as it moves away from the center of the needle body 210, and gradually increases in thickness as it goes from the upper end to the lower end connected to the base film 100. Accordingly, the support wings 250 may have a triangular cross section.

According to the embodiment, the support wings 250 may be provided in a section between the second region 212 and the first region 211. That is, the upper end of the support wings 250 is located at the same height as the maximum width region of the second region 212, and the lower end thereof is connected to the base film 100. Further, the connection region 251 where the lower end of the support wing 250 and the base film 100 are connected may be on the same line as the outer corner portion of the maximum width of the second region 212 when the end thereof is viewed from above.

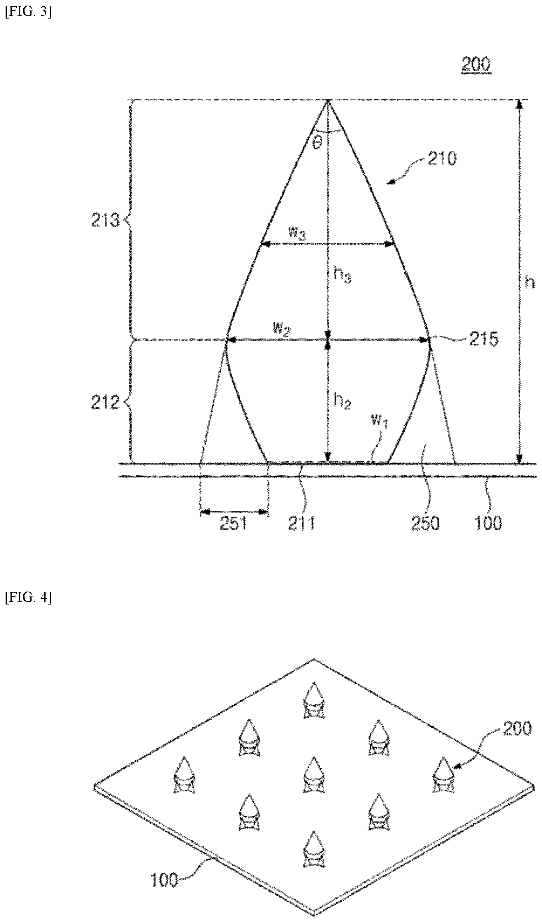

FIG. 3 is a view showing an optimized size of the microneedle according to the embodiment of FIG. 2.

Referring to FIG. 3, the needle body 210 may have a height of 10 µm to 2,000 µm, the second region 212 may have a height h2 of 5 µm to 1,995 µm, and the third region 213 may have a height h3 of 5 µm to 1,995 µm. In addition, the first region 212 may have a widthwise length w1 of 1 µm to 750 µm, and the second region 212 may have a widthwise maximum width w2 of 1.1 µm to 900 µm. The third region 213 may have a widthwise width w3 of 0.1 µm to 500 µm in the middle region from the second region 212 to the end. The third region 213 may have a tip angle θ of 10 degrees to 60 degrees.

The support wings 250 may be provided at a height of 1 µm to 1,000 µm, and a width of a lower end 251 connected to the base film 100 of 0.1 µm to 750 µm. The width from the end of one support wing 250 to the end of the other support wing 250 located at the opposite side of the support body 210 may be 1.1 µm to 1,500 µm.

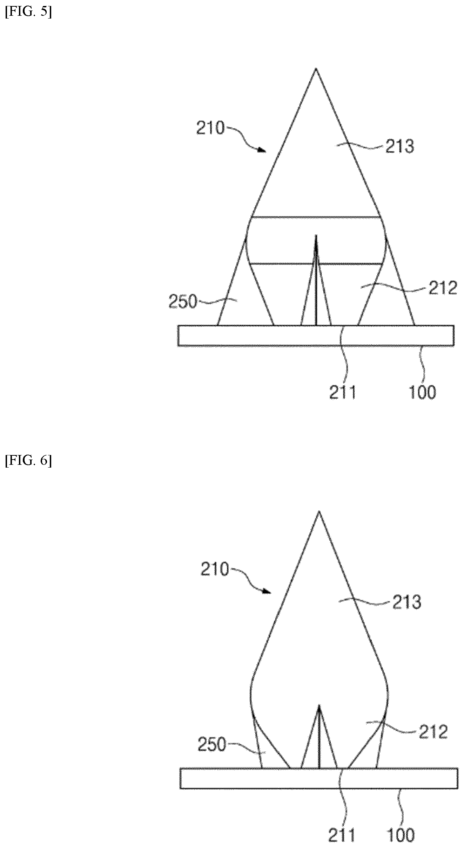

FIG. 4 is a perspective view showing a microstructure according to other embodiment of the present invention, and FIG. 5 is an enlarged view of the microneedle shown in FIG. 4.

Referring to FIGS. 4 and 5, the support wing 250 of the microstructure may be provided so that the length of the lower end thereof is larger than that of the support wing 250 shown in FIG. 2. Accordingly, the length of the region where the support wings 250 and the base film 100 are connected may be provided to be larger than the maximum radius of the second region 212. In addition, the outer corner of the support wing 250 may extend at the same angle as the inclined surface of the third region 213.

FIG. 6 is a view showing a microneedle according to another embodiment of the present invention.

Referring to FIG. 6, an end of a region where the support wing 250 and the base film 100 are connected may be located inside the maximum width of the second region 212 when viewed from above. In addition, the outer corner of the support wing 250 may be formed to be inclined downward inwardly so as to be adjacent to the central axis of the needle body 210 as it goes to the lower end.

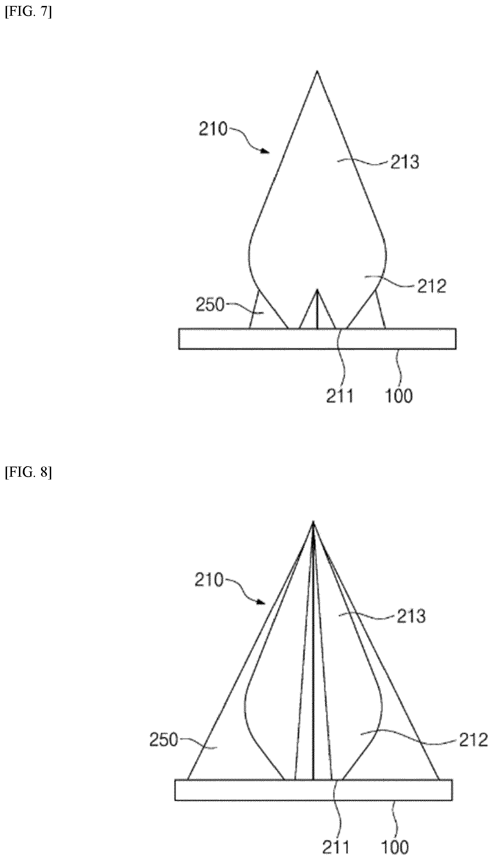

FIG. 7 is a view showing a microneedle according to another embodiment of the present invention.

Referring to FIG. 7, the upper end of the support wing 250 may be located lower than the maximum radius region of the second region 212 .

FIG. 8 is a view showing a microneedle according to another embodiment of the present invention.

Referring to FIG. 8, the support wing 250 extends from the tip region of the needle body 210 and is connected to the base film 100. Due to this, the height from the upper end to the lower end of the support wing 250 is the same as the height of the needle body 210. In addition, the length of the region where the support wing 250 and the base film 100 are connected is provided to be larger than the maximum radius of the second region 212.

As described above, the support wings 250 may be manufactured in various sizes and coupling relationships with the needle body 210.

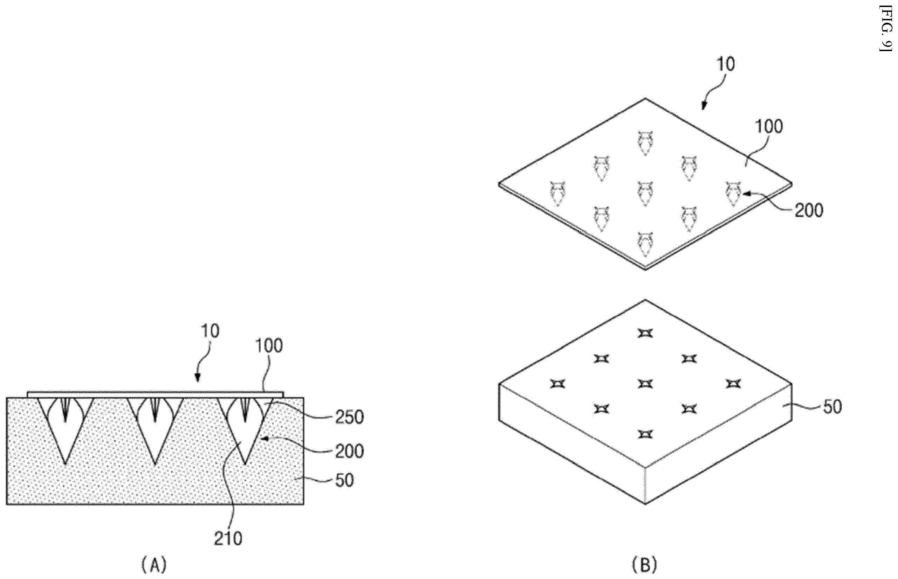

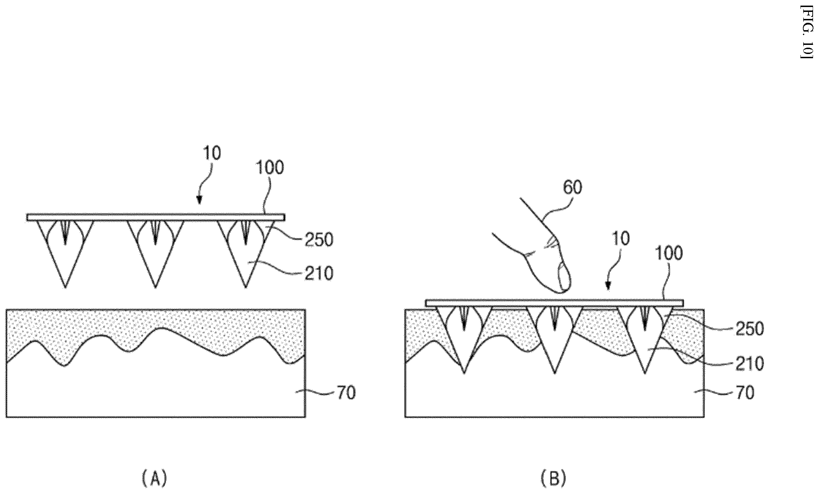

FIG. 9 is a view showing a process of manufacturing a microstructure using a mold, and FIG. 10 is a view showing a process of inserting a microstructure according to an embodiment of the present invention into the skin.

Referring to FIGS. 9 and 10, the support wings 250 increase bonding strength between the needle body 210 and the base film 10. Therefore, the microstructure 10 may be stably separated without damaging the needle body 210 in the process of separating the microstructure 10 from the mold 50. In addition, the needle body 210 may be stably inserted into the skin 70 without damage due to the support of the support wings 250 in the process of infiltrating the needle body 210 into the skin 70 by pressing the base film 10 with the finger 60.

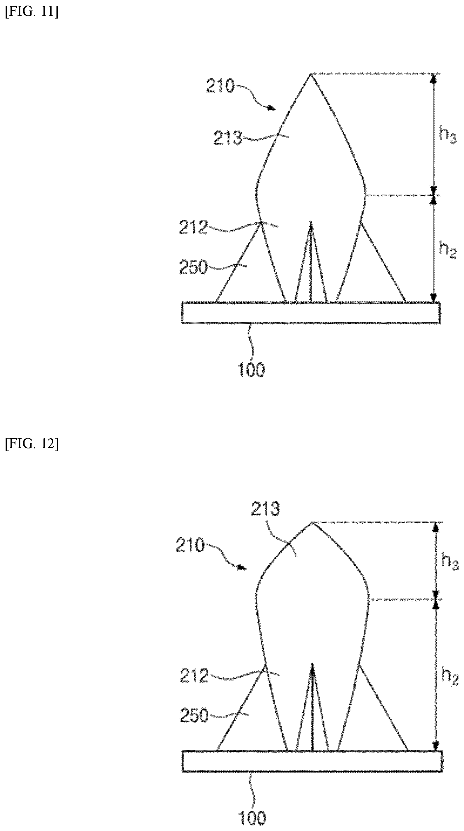

FIG. 11 is a view showing a microneedle according to another embodiment of the present invention.

Referring to FIG. 11, the second region 212 and the third region 213 of the needle body 210 have the same heights h2 and h3. In addition, the support wing 250 is provided in a section between the second region 212 and the first region 211. According to the embodiment, the upper end of the support wing 250 is positioned lower than the maximum radius region of the second region 212. An end of the region where the support wings 250 and the base film 100 are connected protrudes outside the maximum radius region of the second region 212 when viewed from above.

FIG. 12 is a view showing a microneedle according to another embodiment of the present invention.

Referring to FIG. 12, the second region 212 of the needle body 210 is formed to have a large height compared to the third region 213. Due to this, the third region 213 has a large tip angle compared to the third region 213 of the needle body 210 shown in FIG. 3.

In the microstructures described in FIGS. 1 to 12, heights of the maximum radius regions of the second regions 212 of the needle bodies 210 are provided to be different from each other. The heights of the maximum radius regions of the second regions 212 described in FIGS. 1 to 10 are provided to be smaller than those of the third regions 213, the height of the maximum radius region of the second region 212 described in FIG. 11 is provided to be the same as that of the third region 213, and the height of the maximum radius region of the second region 212 described in FIG. 12 is provided to be larger than that of the third region 213. Since such various heights of the maximum radius regions of the second regions 212 are proportional to the skin layer depth at which the microneedles 200 may be interlocked, the drug can be released according to the target skin layer.

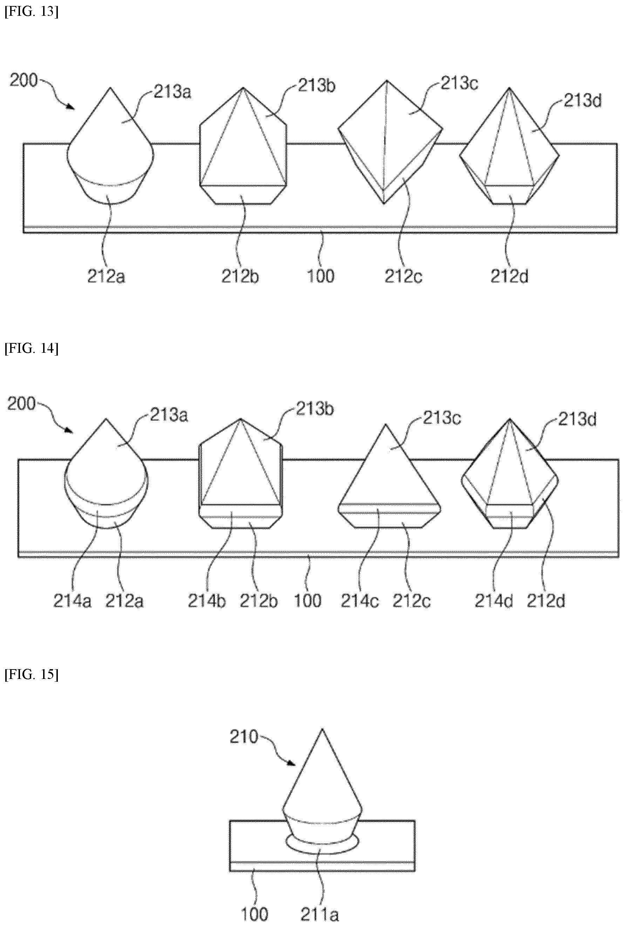

FIGS. 13 and 14 are views showing various shapes of a needle body according to an embodiment of the present invention.

Referring to FIG. 13, the needle body 210 may be provided to have third regions 213a to 213d in various shapes such as a cone, a triangular pyramid, a quadrangular pyramid, and a pentagonal pyramid, and sizes. Such various shapes of the needle body 210 may be selected according to the delivery amount and delivery pattern of the drug.

Referring to FIG. 14, unlike the needle body of FIG. 13 in which the second regions 212a to 212d and the third regions 213a to 213d are connected at a predetermined angle, the needle body of FIG. 14 may have fillets 214a to 214d formed in the connection regions between the second regions 212a to 212d and the third regions 213a to 213d. The fillets 214a to 214d connect the second regions 212a to 212d and the third regions 213a to 213d of the needle body with a curved surface.

FIG. 15 is a view showing a needle body and a base film according to one embodiment of the present invention. For convenience of description, the support wings are not shown.

Referring to FIG. 15, a fillet 211a may be formed along the circumference of the needle body 210 in the first region 211 where the needle body 210 and the base film are connected. The fillet 211a may reinforce the connection region between the needle body 210 and the base film 100 to improve strength of the microneedle 200 together with the support wings 250 described above.

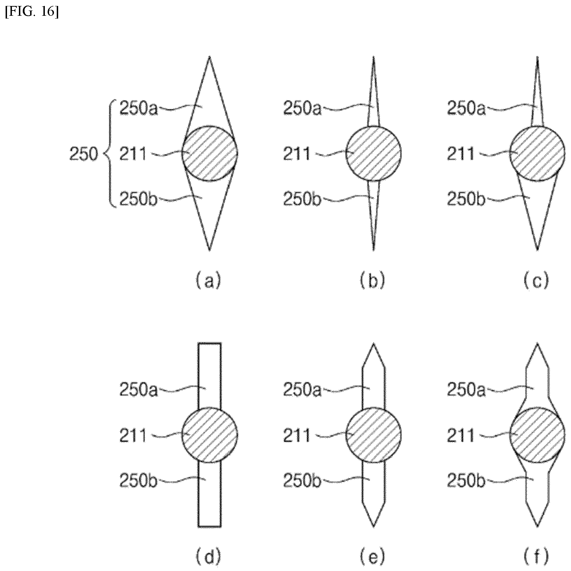

FIG. 16 is a view showing cross sections of support wings according to various embodiments of the present invention. Hereinafter, for convenience of description, the support wing 250 formed on one side of the first region 211 of the support body 210 is referred to as a first support wing 250a, and the support wing 250 formed on the other side thereof is referred to as a second support wing 250b.

Cross sections of the first and second support wings 250a and 250b may have various shapes and sizes. According to the embodiment, the cross sections of the first and second support wings 250a and 250b may have triangular, quadrangular, and pentagonal shapes. The shape and size of the first and second support wings 250a and 250b may be selected depending on the insertion site of the skin, such as the thickness of the skin layer and the hardness of the skin layer.

According to an example, the first and second support wings 250a and 250b may be symmetrical with respect to the first region 211.

According to another example, as shown in FIG. 16C, the first and second support wings 250a and 250b may be asymmetrical with respect to the first region 211. Specifically, the second support wing 250b may be thicker than the first support wing 250a. This is because any one support wing 250b is formed to be thicker than the other support wing 250a along the direction of the load applied to the needle body 210 in the process of separating the microstructure 10 from the mold 50 or inserting it into the skin 70 so that the needle body 210 may be stably supported.

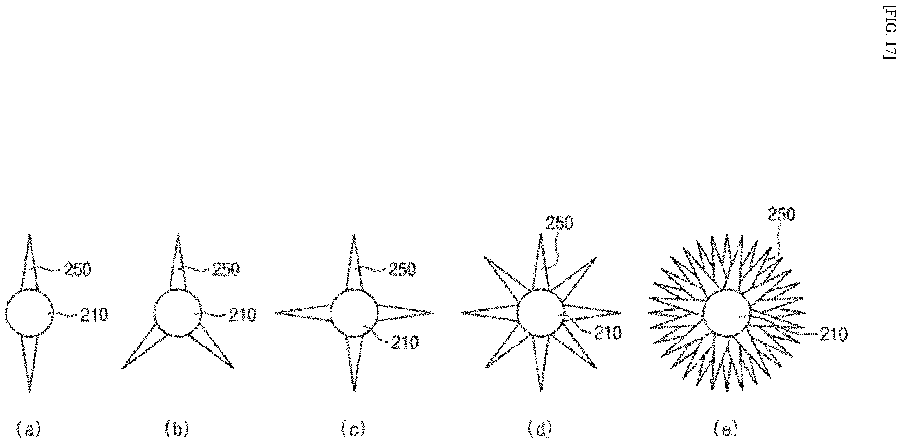

FIG. 17 is a cross-sectional view showing the arrangement of support wings according to various embodiments of the present invention.

Referring to FIG. 17, at least two support wings 250 may be provided depending on the shape and size of the needle body 210, and may be radially disposed around the needle body 210 in various numbers.

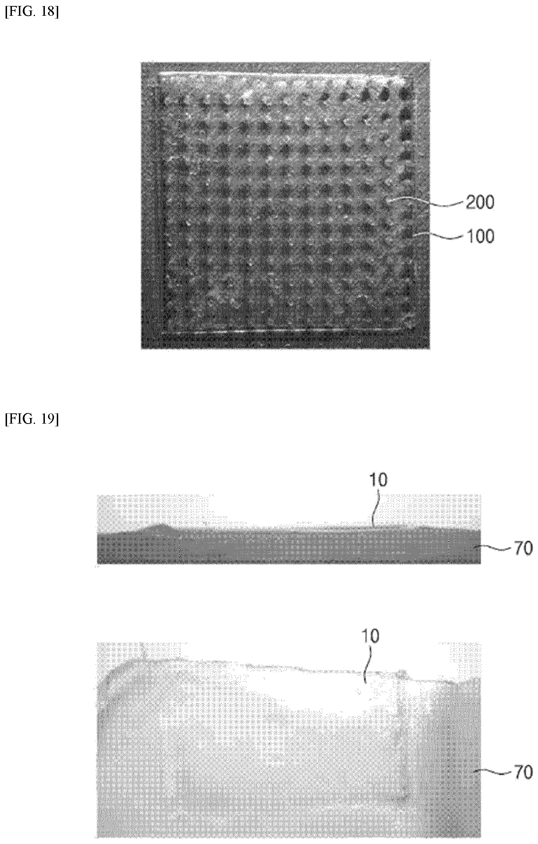

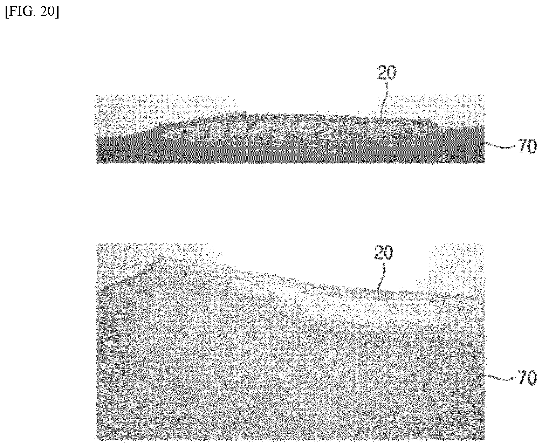

FIG. 18 is an image showing a microstructure and microneedles fabricated according to an embodiment of the present invention, FIG. 19 is a view showing a process of injecting a drug by penetrating the microstructure according to an embodiment of the present invention into the skin, and FIG. 20 is a view showing a process of injecting a drug by penetrating a microstructure according to a comparative example into the skin. In the microstructure 20 according to the comparative example, conical microneedles were used.

FIGS. 18 and 19, due to the shape and the support wings 250 of the needle body 210 according to the embodiment of the present invention, the microneedles 200 may be inserted so that the base film 100 is in close contact with the skin 70. In addition, since the microneedles 200 are interlocked to the skin 70, the microstructure 10 may stably maintain close a state of contact with the skin. After about 30 minutes have elapsed after the insertion of the microstructure 10, it can be confirmed that the drug penetrates the skin and the microstructure 10 is completely decomposed.

Meanwhile, referring to FIG. 20, the cone-shaped microneedles have weak penetrating power into the skin 70, and the penetrated state, that is, interlocking is not stably maintained, so that the base film does not completely adhere to the skin. Therefore, even after 30 minutes have elapsed, it can be seen that the skin penetration efficiency of the drug is low since the microneedles and the base film are remained in the microstructure 20 as they are.

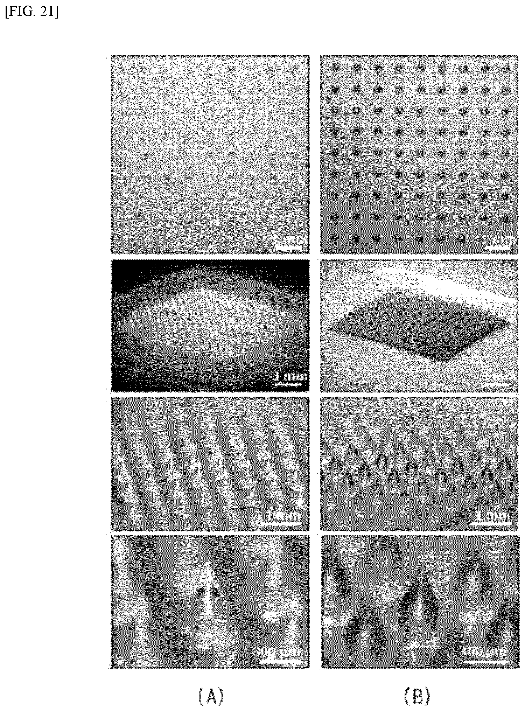

FIG. 21 is a view showing a microstructure fabricated according to one embodiment of the present invention and microneedles thereof.

Referring to FIG. 21, the base film and the microneedles may be made of the same material. FIG. 21A shows a microstructure in which a base film and microneedles are made of hyaluronic acid, and FIG. 21B shows a microstructure in which a base film and microneedles are made by mixing a blue-based dye and hyaluronic acid. In this way, the microstructure can be manufactured in such a manner so that the drug is loaded on the entire area of the base film and the microneedles.

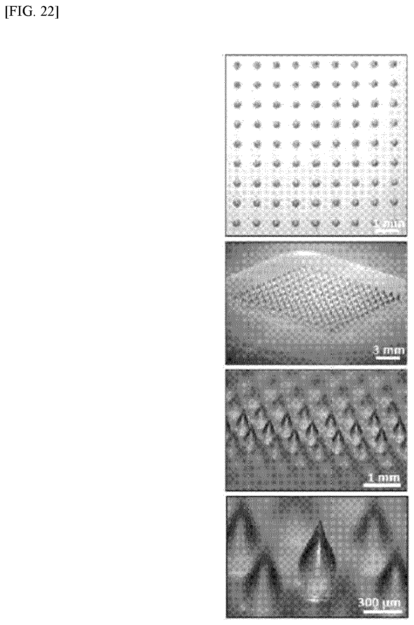

FIG. 22 is a view showing a microstructure fabricated according to other embodiment of the present invention and microneedles thereof.

Referring to FIG. 22, the base film and the microneedles may be made of different materials. When a drug is loaded onto the base film, there is a possibility that the drug may not be delivered to the skin, and in this case, it is required that the drug be selectively loaded onto the microneedle. The drug may be loaded onto the entireties or tip portions of the microneedles. In the microneedles shown in FIG. 22, a blue-based dye is loaded onto the tip portions thereof. In this way, it is possible to selectively load the drug onto the tip portions of the microneedles.

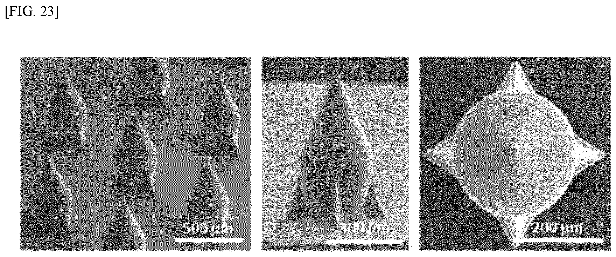

FIG. 23 is an enlarged view of a microneedle fabricated according to an embodiment of the present invention.

Hereinabove, the present invention has been described in detail using preferred embodiments, but the scope of the present invention is not limited to specific embodiments, and should be interpreted according to the appended claims. In addition, those skilled in the art should understand that many modifications and variations are possible without departing from the scope of the present invention.

### [Industrial Applicability]

The microstructure according to the present invention can be used for medical treatment and skin care.

## Claims

1. A microstructure comprising:
a base film; and
a plurality of microneedles formed on one surface of the base film,
wherein the microneedle comprises:
a needle body; and
support wings which are disposed in a plurality around the needle body, connect the outer surface of the needle body and the base film, and have a thickness thinner than that of the needle body.

2. The microstructure of claim 1, wherein the support wings are symmetrical about the center of the needle body.

3. The microstructure of claim 1, wherein the support wings gradually decrease in thickness as they are away from the center of the needle body.

4. The microstructure of claim 1, wherein the needle body comprises:
a first region coupled to the base film;
a second region extending from the first region and gradually increasing in width as the distance from the first region increases; and
a third region extending from the second region and gradually decreasing in width as it goes to an end thereof, and
each of the support wings is provided in a section between the first region and the second region.

5. The microstructure of claim 4, wherein the region where the support wings and the base film are connected has an end located inside the maximum radius region of the second region when viewed from above.

6. The microstructure of claim 4, wherein the region where the support wings and the base film are connected has an end located on the same line as the maximum radius region of the second region when viewed from above.

7. The microstructure of claim 4, wherein the region where the support wings and the base film are connected has an end located outside the maximum radius region of the second region when viewed from above.

8. The microstructure of claim 4, wherein the connection region between the second region and the third region has an outer circumferential surface provided as a curved surface.

9. The microstructure of claim 1, wherein the needle body comprises:
a first region coupled to the base film;
a second region extending from the first region and gradually increasing in width as the distance from the first region increases; and
a third region extending from the second region and gradually decreasing in width as it goes to an end thereof, and
each of the support wings extends downward from an end of the third region and is connected to the base film.

10. The microstructure of claim 9, wherein the connection region where the support wings and the base film are connected is larger than a maximum radius of the second region.

11. The microstructure of claim 1, wherein the support wings including:
a first support wing 250 located on one side of the needle body; and
a second support wing located on the opposite side of the first support wing centering around the needle body,
and the first support wing and the second support wing have different thicknesses.

12. The microstructure of claim 1, wherein the support wings gradually increase in thickness as it goes from an upper end thereof to a lower end adjacent to the base film.
